# EUROPEAN PATENT SPECIFICATION

(11) **EP 1 915 907 B1**
(45) Date of publication and mention of the grant of the patent: **17.11.2010**
(21) Application number: 08001427.7
(22) Date of filing: 26.03.1996
(51) Int. Cl.: A01N 43/828

(54) **An agricultural and horticultural disease controller**
Schädlingsbekämpfungsmittel für Landwirtschaft und Gartenbau
Agent de lutte contre les maladies dans les domaines de l'agriculture et de l'horticulture

(30) Priority: 31.03.1995 JP 9988095
(43) Date of publication of application: 30.04.2008
(62) Divisional of application: 06008951.3
(73) Proprietor: NIHON NOHYAKU CO., LTD., Tokyo 103-8236 (JP)
(72) Inventor: Kuroda, Kiyoshi, Osaka, 567-0892 (JP); Tajima, Sokichi c/o Nihon Nohyaku Co., Ltd., Tokyo 103-8236 (JP); Uchikurochane, Toru, Hashimoto-shi, Wakayama 648 (JP); Tsubata, Kenji, Kawachinagano-shi, Osaka 586 (JP)
(74) Representative: Grünecker, Kinkeldey, Stockmair & Schwanhäusser Anwaltssozietät

(56) References cited:
- EP-A- 0 046 497
- FR-A- 2 395 263
- FR-A- 2 451 371
- FR-A- 2 453 165
- US-A- 4 177 054

## Description

The present invention relates to a 1, 2, 3-thiadiazole derivative represented by the formula (1), or a salt thereof:

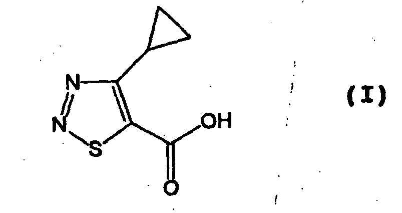

Japanese Patent Unexamined Publication No. 54-9272 discloses 1,2,3-thiadiazole-5-carboxylic acid derivatives, a process for production thereof and an agent containing the derivative and having herbicidal and growth-regulating effects.

The present inventors earnestly investigated for developing a novel agricultural and horticultural disease controller and consequently found that the 1,2,3-thiadiazole derivative of formula (I) or salts thereof is useful for herbicide and growth regulater, whereby the present invention has been accomplished.

As the salt of the 1,2,3-thiadiazole derivative of the formula (I), there can be exemplified salts with alkali metals such as sodium, potassium, etc.; salts with alkaline earth metals such as calcium, magnesium, etc.; unsubstituted ammonium salt; substituted ammonium salts having one or more substituents which may be the same or different and are selected from the group consisting of (C₁-C₁₂)alkyl groups, unsubstituted phenyl group, substituted phenyl groups, unsubstituted benzyl group and substituted benzyl groups; and guanidium salt.

The 1,2,3-thiadiazole derivative of the formula (I) or salt thereof used as the active ingredient of the agricultural and horticultural disease controller of the present invention can be produced, for example, by any of the processes exemplified below: 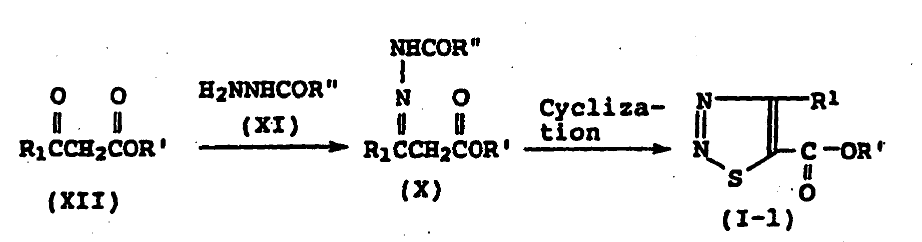 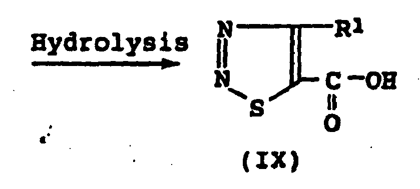 wherein R¹ is, R' is a (C₁-C₆)alkyl group and R" is an amino group or a (C₁-C₆)alkyl group.

A compound of the general formula (XII) is reacted with a compound of the general formula (XI) to obtain a compound of the general formula (X). The compound (X) is cyclized after or without isolation to obtain a 1,2,3-thiadiazole derivative of the general formula (I-1). The derivative (I-1) is hydrolyzed after or without isolation to obtain a compound of the general formula (IX).

The compound of the general formula (XII) can be produced by the process described in J. Org. Chem., 43, 2087 (1978), Formation of C-C bonds, Vol. 3, p. 259, 1979 (Georg Thime Publishers, Stuttgart), etc.

The compound of the formula (I) or a salt thereof has a melting point of 157.1°C.

The 1,2,3-thiadiazole derivative of the formula (I) or salts thereof according to the present invention is useful for agricultural and horticultural disease control. For example, the compound is very effective in controlling various diseases, for instance, rice blast (Pyricularia oryzae), rice sheath blight (Rhizoctonia solani), rice helminthosporium leaf spot (Cochliobolus miyabeanus), powdery mildew of various host plants, such as powdery mildew of barley and wheat (Erysiphe graminis), oats crown rust (Puccinia coronata), rust of other plants, tomato late blight (Phytophthora infestans), late blight or Phytophthora rots of other plants, downy mildew of various plants, such as cucumber downy mildew (Pseudoperonospora cubensis) and grape downy mildew (Plasmopara viticola), apple scab (Venturia inaequalis), apple alternaria leaf spot (Alternaria mali), pear black spot (Alternaria kikuchiana), citrus melanose (Diaporthe citri), cucumber bacterial blight (Pseudomonas syringae pv. lachrymans), tomato bacterial wilt (Pseudomonas solanacearum), cabbage black rot (Xanthomonas campestris), citrus canker (Xanthomonas citri (Hasse) Dowson), rice bacterial leaf blight (Xanthomonas oryzae), cabbage bacterial soft rot (Erwinia carotovora), and tobacco mosaic (Tobacco mosaic virus).

The agricultural and horticultural disease controller of the present invention is markedly effective in controlling the above-exemplified diseases which damage paddy field crops, upland crops, fruit trees, vegetables, other crops, flowers and ornamental plants, and the like. Therefore, the desired effects of the agricultural and horticultural disease controller of the present invention can be obtained by applying the disease controller to the paddy field water, stalks and leaves of fruit trees, vegetables, other crops, flowers and ornamental plants, soil, etc., at a season at which the diseases are expected to occur, before their occurrence or at the time when their occurrence is confirmed.

In general, the agricultural and horticultural disease controller of the present invention is used after being prepared into a conveniently usable form according to an ordinary manner for preparation of agrochemicals.

That is, the 1,2,3-thiadiazole derivative of the formula (I) or salt thereof according to the present invention and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in the present invention may be either solid or liquid. As the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain calcium silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphta and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The present inventive agricultural and horticultural disease controller containing the 1,2,3-thiadiazole derivative of the formula (I) or a salt thereof as an active ingredient is used to control various diseases in the following manner. That is, it is applied to a crop on which the diseases are expected to occur, or a site where the occurrence of the diseases is undesirable, as it is or after being properly diluted with or suspended in water or the like, in an amount effective for control of the diseases. For example, to control the diseases of paddy rice, said disease controller can be used by a method such as submerged application to a regular paddy field, application to a rice nursery bed, dressing of seeds for direct sowing on flooded paddy field, or seed disinfection.

The applying dosage of the present inventive agricultural and horticultural disease controller containing the 1,2,3-thiadiazole derivative of the formula (I) or a salt thereof as an active ingredient is varied depending upon various factors such as a purpose, diseases to be controlled, a growth state of a plant, tendency of disease occurrence, weather, environmental conditions, a preparation form, an application method, an application site and application time. It may be properly chosen in the range of 0.1 g to 10 kg (in terms of the active ingredient) per 10 ares depending upon purposes.

The present inventive agricultural and horticultural disease controller containing the 1,2,3-thiadiazole derivative of the formula (I) or a salt thereof as an active ingredient may be used in admixture with other agricultural and horticultural disease controllers in order to expand both spectrum of controllable diseases and the period of time when effective applications are possible or to reduce the dosage.

Typical examples and test examples of the present invention are described below but they should not be construed as limiting the scope of the invention.

In the examples, parts are all by weight.

### Example 1

| | |
|---|---|
| The compound of formula (I) | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

### Example 2

| | |
|---|---|
| The compound of formula (I) | 3 parts |
| Clay powder | 82 parts |
| Diatomaceous earth powder | 15 parts |

A dust was prepared by mixing uniformly and grinding the above ingredients.

### Example 3

| | |
|---|---|
| The compound of formula (I) | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

### Example 4

| | |
|---|---|
| The compound of formula (I) | 20 parts |
| Mixture of kaolin and synthetic, high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

### Test Example 1

### Controlling effect on rice blast by submerged application

Rice plants at the 5 to 6 leaf stage cultivated in a 1/10000-are pot were subjected to submerged application of a chemical containing the compound of formula (I) as an active ingredient, in a dosage of 200 g/10 a in terms of the active ingredient. After standing in a greenhouse for 1 week, the plants were inoculated with a suspension of spores of blast fungus (Pyricularia oryzae) by spraying.

After the inoculation, the plants were allowed to stand in a moist chamber for 1 day and then a greenhouse for 6 days to cause the disease sufficiently. Then, lesions in each leaf were counted and then compared with those on the untreated plot, and the controlling degree was calculated, whereby the effect was judged according to the following criterion.

| Effect | Controlling degree (%) |
|---|---|
| A | 100 - 95 |
| B | 94 - 85 |
| C | 84 - 60 |
| D | 59 - 0 |

As a result of the above test, the compound of formula (I) was found to have a marked blast-controlling activity. In particular, the compound of formula (I) was rated A, namely, it had an excellent blast-controlling activity:

## Claims

1. A compound according to formula (I) 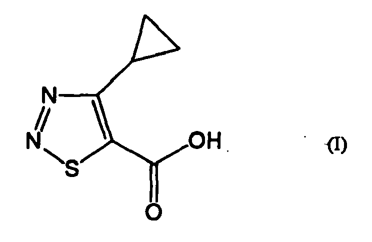 or a salt thereof.

## Patentansprüche

1. Verbindung gemäß Formel (I) 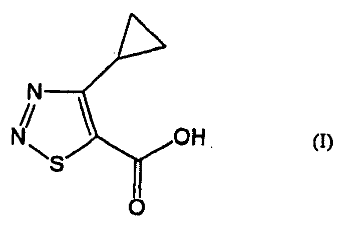 oder ein Salz davon.

## Revendications

1. Composé selon la formule (I) 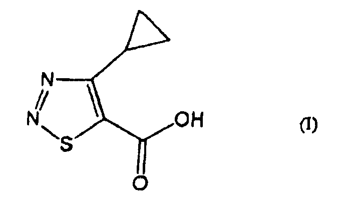 ou un sel de celui-ci.
